# EUROPÄISCHE PATENTANMELDUNG

(11) **EP 0 741 293 A2**
(43) Veröffentlichungstag der Anmeldung: **06.11.1996**
(21) Anmeldenummer: 96103239.8
(22) Anmeldetag: 02.03.1996
(51) Int. Cl.: G01N 30/12, G01N 31/12

(54) **Verfahren zur Untersuchung silanbehandelter, anorganischer Materialien**

(30) Priorität: 13.04.1995 DE 19514033
(71) Anmelder: HÜLS AKTIENGESELLSCHAFT, D-45764 Marl (DE)
(72) Erfinder: Rotzsche, Harald, Dr., 83451 Piding (DE); Ditscheid, Klaus-Peter, 53840 Troisdorf (DE)

(57) **Zusammenfassung**

Die Erfindung betrifft ein Verfahren zur Untersuchung von anorganischen Materialien, die mit siliziumorganischen Verbindungen behandelt sind, wobei das Probenmaterial innerhalb weniger Sekunden pyrolysiert wird und die Pyrolyseprodukte on-line gaschromatographisch an einer PLOT-Säule (porous layer open-tubular -Säule) analysiert werden.

## Beschreibung

Die Erfindung betrifft ein Verfahren zur Untersuchung von anorganischen Materialien, die mit siliziumorganischen Verbindungen behandelt sind.

Es ist bekannt, daß die Hydrophobierung anorganischer Materialien, insbesondere die mineralischer Baustoffe, in der Regel mit siliziumorganischen Verbindungen in Form wäßriger Emulsionen, Mikroemulsionen oder gelöst in organischen Lösemitteln vorgenommen werden (Produktinformationen der Hüls AG: "Anwendungen von organofunktionellen Silanen - DYNASYLAN®", 15.01.002/07.94/gu/100; "Bautenschutz mit Alkylsilanen - DYNASYLAN® BSM", 15.01.006/09.90/gu; Ch. Fliedner, "Silane als Hydrophobierungsmittel für Beton", Sonderdruck 15.07.023 der Hüls AG, Teile I und II sind erschienen in: Bautenschutz + Bausanierung 3/94 und 4/94). Auf der Oberfläche der anorganischen Materialien, bis in das Innere eines porösen Stoffes, gehen reaktionsfähige Gruppen der Organosiliziumverbindungen chemische Bindungen mit den Hydroxylgruppen des Stoffes ein; parallel dazu laufen in Gegenwart von Wasser Vernetzungsreaktionen der Siliziumverbindungen ab, was schließlich zu einem auf der Oberfläche und auch in den Poren chemisch verankerten hydrophobierenden Film führt, der die zerstörerische Aufnahme und den Transport von Wasser unterbindet oder reduziert. Von den Baustoffen seien hier beispielsweise Beton, Sandstein, Kalksandstein, Ziegel und Mörtel erwähnt. Auch pulverförmige und körnige Materialien wie Aluminiumoxid, Titandioxid, Magnesiumoxid, Glas- und Quarzpulver, Hydroxide, Silikate und andere mineralische Produkte sowie Mikroglaskügelchen werden in erheblichem Umfang mit Organosiliziumverbindungen oberflächenmodifiziert, um ihre anwendungstechnischen Eigenschaften zu verbessern.

Gegenwärtig erfolgt die Prüfung eines Oberflächenschutzes von Baustoffen an Hand der Wasseraufnahme (Technische Prüfvorschrift Oberflächenschutzsysteme, Ausgabe 1990, Verkehrsblatt, Dokument-Nr.: B 5234 - Vers. 90.1, Seite 30), der Imprägniertiefe (Freilegung einer Stelle bis ≧ 1 cm und Besprühen mit Wasser), des Saugprofils (Wasseraufnahme an nacheinander abgesägten Scheiben, M. Roth: Das Wassersaugprofil einer Siliconimprägnierung, Bautenschutz und Bausanierung, 11, (1988), Seiten 43 - 45), der Druckwasserlagerung (DIN 52 103) oder der Saugwasserlagerung (DIN 52 617). Entscheidend für die Qualität der Hydrophobierung sind die chemische Struktur des Hydrophobierungsmittels, die aufgenommene Wirkstoffmenge an der Oberfläche und in tieferen Zonen und die chemische Verankerung des Hydrophobierungsmittels auf den inneren und äußeren Oberflächen des Baustoffes; hierzu können mit den oben aufgeführten Prüfungen praktisch keine Aussagen erhalten werden, da nur pauschal der wasserabweisende Effekt gemessen wird.

Für die Untersuchung von Oberflächen wurden verschiedene Analysenmethoden, wie TOF-SIMS (Sekundärionen-Flugzeitmassenspektrometrie), ESCA (Elektronenspektroskopie zur chemischen Analyse), DRIFT (Diffuse Reflections-Infrarot-Fourier-Transformation) und Auger-Elektronenspektroskopie, entwickelt. Abgesehen von einem hohen Geräte- und Zeitaufwand sowie enormen Kosten, haben diese Methoden den Nachteil, daß meist nur die oberste Oberfläche des Probenmaterials im Nanometerbereich erfaßbar ist.

Zur Charakterisierung von Silan-Hydrophobierungsmitteln unter praxisnahen Bedingungen ist auch die dispersive IR(Infrarotspektroskopie)-Technik nicht geeignet (Ghosh, S. und Handoo, S. K.: Infrared and Raman Spectral Studies in Cement and Concrete, Cement and Concrete Research, 10, (1980), Seiten 771 - 782). Allerdings konnte die Eindringtiefe von Hydrophobierungsmitteln in Beton durch FT(Fourier-Transformations)-IR-Spektroskopie bestimmt werden (A. Gerdes, T. Müller und F. H. Wittmann in: Werkstoffwissenschaften und Bausanierung, Teil 1, Tagungsbericht des 3. Internat. Kolloqu., Hrsg. F. H. Wittmann, expert-Verlag (Kontakt & Studium Bd. 420) Ehningen bei Böblingen, 1993, Seiten 460 - 475). Analysenfein gemahlenes Betonmehl wurde mit Kaliumbromid gemischt (1 : 25) und bei 250 bar zu durchsichtigen Tabletten gepreßt und daran im Absorptionsbereich von 2 900 - 2 940 cm⁻¹ ein FT-IR-Spektrogramme aufgenommen. Die CH₂-Valenzschwingung im Bereich 2 930 cm⁻¹ diente zur qualitativen bzw. halbquantitativen Erfassung von Hydrophobierungsmitteln auf Organosiliziumbasis, vorausgesetzt, daß organische Verbindungen, die den Nachweis stören können, nicht zugegen sind. Abgesehen vom Geräte- und Präparationsaufwand weist diese Methode weitere Nachteile auf: Zwischen den verschiedenen Hydrophobierungs- bzw. Modifizierungsmitteln kann nicht unterschieden werden; Wirkstoffgehalte unter etwa 0,2 % sind nicht erfaßbar; grobe Partikel sind ohne Feinmahlung nicht analysierbar; Hydrophobierungsmittelgemische sind als solche nicht erkennbar; zwischen kovalenter Fixierung und physikalischer Belegung kann nicht unterschieden werden; Modifizierungen von z. B. Glasperlen, Aluminiumoxid oder Silikaten sind mangels Empfindlichkeit der Methode nicht erkennbar.

Auch die ²⁹Si-MAS-NMR-Spektroskopie (29-Si-magic-angle-spinning-Kernresonanzspektroskopie) ist wegen des hohen Kostenaufwandes und der begrenzten Nachweisempfindlichkeit für eine Qualitätsprüfung bei Anwendung der Hydrophobierungs- und Modifizierungsmittel in der täglichen Praxis nicht geeignet, - diese Methode wird eher für Grundlagenuntersuchungen eingesetzt (J. Grobe, K. Stoppek-Langner, W. Müller-Warmuth, S. Thomas, A. Benninghoven und B. Hagenhoff: Nachr. Chem. Tech. Lab., 41, (1993), Nr. 11, Seiten 1233 - 1240; B. Hagenhoff, A. Benninghoven, K. Stoppek-Langner und J. Grobe: Adv. Mater., (1994), 6, No. 2, Seiten 142 - 144; K. Albert, R. Brindle, J. Schmid, B. Buszewski und E. Bayer: Chromatographia, 38, (1994), Seiten 283 - 290).

Die Pyrolyse von silanbeschichteten Füllstoffen, speziell Talkum, wurde zum Nachweis von zur Beschichtung eingesetzten organofunktionellen Silanen genutzt, indem eine relativ große Menge Füllstoff (10 g) bei 720 °C etwa 30 Minuten lang pyrolysiert wurde; die große Menge war erforderlich, um die Nachweisgrenzen von 0,01 - 0,1 % zu erreichen und die lange Pyrolysedauer verbunden mit der "großen" Probenmenge, um Sekundärreaktionen (Artefaktbildung) zu begünstigen, die die Unterscheidung einzelner Silane erleichtern sollen. Die Pyrolyseprodukte wurden in einem organischen Lösemittel (Triethylenglykoldimethylether) bei 0 °C aufgefangen und die Absorptionslösung zusammen mit dem Kondensat, das sich vor dem Absorber abscheiden kann, anschließend gaschromatographisch (off-line) analysiert, Trennsäule: 60 m DB-Wax Dünnfilmkapillare, Filmdicke: 0,5 µm (R. Doiber und N. Wamser: Fresenius J. Anal. Chem., 342, (1992), Seiten 381 - 386). Nachteilig bei diesem Verfahren ist es, daß nur in Ausnahmefällen Reaktionsprodukte entstehen, die charakteristisch oder selektiv für bestimmte Beschichtungsmittel sind (Ammoniak, Schwefelwasserstoff, Aceton), und daß das Gros der zahlreichen unüberschaubaren Pyrolyseprodukte für alle Silane identisch ist, so daß nur quantitative Unterschiede in der gaschromatographischen Peakverteilung zur Identifizierung herangezogen werden können. Auch können die für viele Hydrophobierungs- und Modifizierungsmittel charakteristischen Alkoxygruppen (Methoxy- oder Ethoxygruppen) nicht nachgewiesen werden.

Die sehr aufwendige und darüber hinaus kostenintensive FT-IR-Analysenmethode (Pyrolyse-Gaschromatographie mit Fourier-Transformations-Infrarotspektroskopie) wurde eingesetzt, um festzustellen, ob N-Phenyl-1-aminopropyltrimethoxysilan in Essigsäure bei der Silanbehandlung von E-Glasfasern chemisch gebunden bzw. mit Methanol abwaschbar ist (N. Ikuta, T. Hori, H. Naitoh, Y. Kera, E. Nishio und I. Abe, Compos. Interfaces (1993), 1(6), 455 - 462).

Mittels Pyrolyse-Gaschromatographie wurden auch Poly(diorganosiloxane) untersucht (S. Fujimoto, H. Ohtani und S. Tsuge: Fresenius Z. Anal. Chem., 331, (1988), Seiten 342 - 350), wobei in der Hauptsache eine Vielzahl cyclischer Oligomerer entstanden, die an einer Dünnfilmkapillare (WCOT-Säule) getrennt wurden und deren quantitative Analyse eine Bestimmung der ursprünglichen Zusammensetzung anhand der oft mehr als 19 Komponenten nur mit einem aufwendigen Verfahren ermöglichte.

Zur Qualitätsbeurteilung der Hydrophobierung bzw. Oberflächenmodifikation anorganischer Materialien besteht ein Bedarf für ein Untersuchungsverfahren, mit dem die hier verwendeten Organosiliziumverbindungen möglichst spezifisch nachgewiesen, ihre Konzentration auf der Oberfläche und ggf. auch in verschiedenen Tiefen des anorganischen Substrats quantitativ bestimmt werden kann.

Der Erfindung lag daher die Aufgabe zugrunde, ein Verfahren bereitzustellen, das es ermöglicht, anorganische Materialien, die zur Hydrophobierung bzw. Oberflächenmodifizierung mit siliziumorganischen Verbindungen behandelt wurden, mit vertretbarem Aufwand zu untersuchen, die eingesetzten Organosiliziumverbindungen möglichst sicher zu identifizieren und auch quantifizieren zu können.

Es wurde überraschenderweise gefunden, daß siliziumorganische Hydrophobierungs- und Modifizierungsmittel auf anorganischen Materialien, insbesondere auf und in Baustoffen, spezifisch nachgewiesen und quantitativ reproduzierbar bestimmt werden können, indem man im allgemeinen das Probenmaterial zunächst innerhalb weniger Sekunden pyrolysiert und die Pyrolyseprodukte on-line gaschromatographisch an Dünnschichtkapillaren (PLOT-Säulen - porous layer open-tubular -Säulen, d. h. Kapillarsäule und poröser Schicht) analysiert. Geeigneterweise nutzt man eine bei der Pyrolyse bevorzugt gebildete Hauptkomponente zur Identifizierung und quantitativen Bestimmung.

Gegenstand der vorliegenden Erfindung ist daher ein Verfahren zur Untersuchung von anorganischen Materialien, die mit siliziumorganischen Verbindungen behandelt sind, das dadurch gekennzeichnet ist, daß das Probenmaterial innerhalb weniger Sekunden pyrolysiert wird und die Pyrolyseprodukte on-line gaschromatographisch an einer PLOT-Säule (porous layer open-tubular -Säule, d. h. Kapillarsäule mit poröser Schicht) analysiert werden.

Geeigneterweise wird im erfindungsgemäßen Verfahren die Identifizierung der Organosiliziumverbindungen, vorzugsweise auch deren quantitative Bestimmung, anhand von Komponenten, d. h. solchen Pyrolyseprodukten, die unter den vorherrschenden Pyrolysebedingungen bevorzugt gebildet werden, durchgeführt.

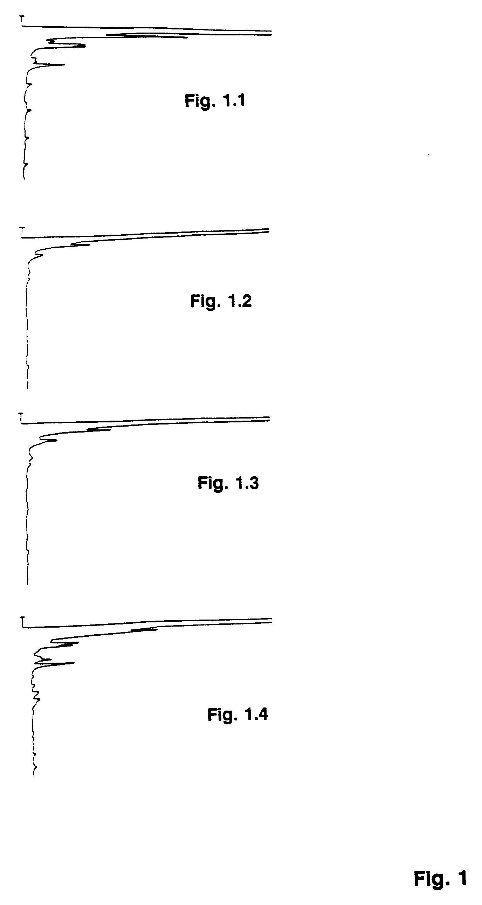

Fig. 1 zeigt die Pyrolyseprodukte von vier Hydrophobierungsmitteln (Methyl-, Ethyl-, Propyl- und Isobutyltriethoxysilan), die jeweils auf Beton mit 0,4 Gew.-% aufgebracht und auf herkömmliche Weise pyrolytisch-gaschromatographisch untersucht wurden (Dünnfilmkapillare, WCOT-Säule). Die Unterschiede zwischen den einzelnen Bautenschutzmitteln sind nur geringfügig und für eine Identifizierung und Unterscheidung solcher Silanbeschichtungen ungeeignet.

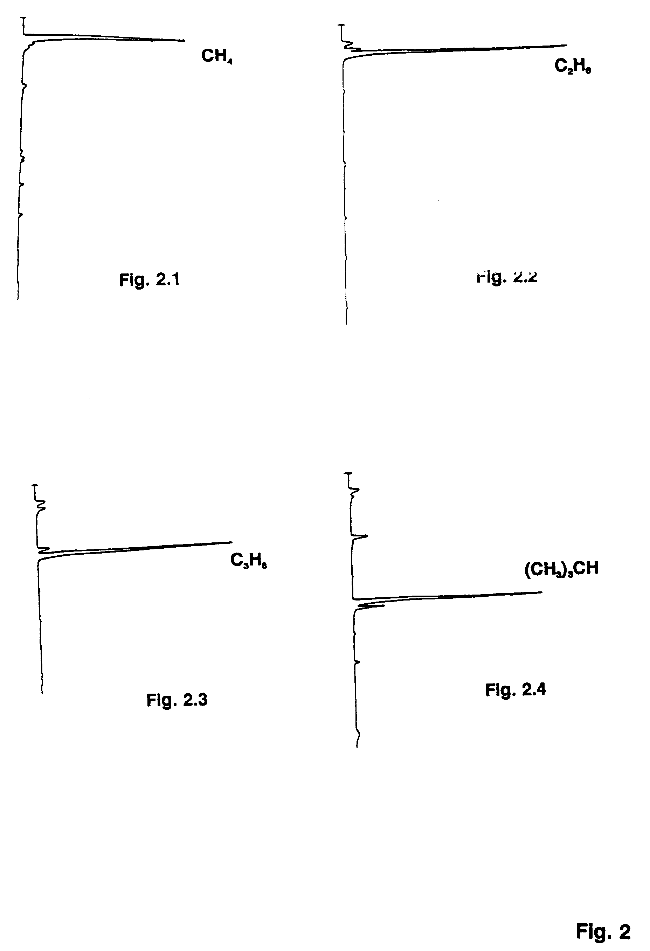

Fig. 2 zeigt die Pyrolyse-Gaschromatogramme der gleichen Proben, wie sie auch bei Untersuchungen für Fig. 1 verwendet wurden, die aber nach dem erfindungsgemäßen Verfahren untersucht wurden; hierfür wurde geeigneterweise eine PLOT-Säule 1 - PoraPLOT Q, 10 m Länge, innerer Durchmesser 0,32 mm, Schichtdicke 10 µm (Chrompack GmbH, Frankfurt/Main) eingesetzt. Überraschend ist dabei, daß Methyltriethoxysilan auf Beton als Pyrolyseprodukt praktisch nur Methan liefert, Ethyltriethoxysilan nur Ethan, Propyltriethoxysilan in der Hauptsache Propan und Isobutyltriethoxysilan vorwiegend Isobutan, obwohl nach der ²⁹Si-NMR-Untersuchung bekannt ist, daß auf hydrophobierten Baustoffoberflächen infolge von Hydrolyse und Polykondensation des monomeren Bautenschutzmittels vorwiegend polymere Netzwerke aus mono-, di- und trifunktionellen Polysiloxanen existieren (J. Grobe et al., Nachr. Chem. Techn. Lab, 41, (1993), Nr. 11, Seiten 1233 - 1240) und die Vielzahl von chemischen Oberflächenstrukturen nicht zum gleichen Pyrolyseprodukt führen sollte.

Die Pyrolysedauer des Probenmaterials beträgt im erfindungsgemäßen Verfahren im allgemeinen weniger als 10 Sekunden, vorzugsweise 8 bis 0,001 Sekunden, besonders vorzugsweise 5 bis 0,1 Sekunden.

Geeigneterweise setzt man beim erfindungsgemäßen Verfahren für die Pyrolyse des Probenmaterials z. B. einen Heizdrahtpyrolysator, einen Curie-Punkt-Pyrolysator, einen Laserpyrolysator oder einen Mikrowellenpyrolysator oder ein anderes Verfahren für den Energieeintrag ein. Bevorzugt wird im erfindungsgemäßen Verfahren ein Schiffchenpyrolysator eingesetzt.

Im erfindungsgemäßen Verfahren werden die Pyrolyseprodukte on-line gaschromatographisch an einer PLOT-Säule, bevorzugt ohne jeglichen Zwischenschritt, wie Adsorption, Kühlfallentechnik, Tieftemperaturfokussierung, Säulenumschaltungstechnik, getrennt und im allgemeinen durch einen Detektor, vorzugsweise durch einen Flammenionisationsdetektor, angezeigt.

Für die gaschromatographische Untersuchung der Pyrolyseprodukte werden im erfindungsgemäßen Verfahren PLOT-Säulen eingesetzt, die vorzugsweise Aluminiumoxid, desaktiviert durch KCl oder Na₂SO₄, oder Silicagel oder graphitierten, thermischen Ruß oder organische Polymere auf Basis Styrol/Divinylbenzol als feste poröse Schicht enthalten. Solche Kapillarsäulen sind in der Regel Quarzkapillaren oder geeignete Metallkapillaren mit einer porösen Schicht aus einem der zuvor genannten Materialien.

Die Pyrolyse des Probenmaterials wird im erfindungsgemäßen Verfahren vorzugsweise im Temperaturbereich zwischen 800 und 500 °C durchgeführt, kann aber auch im Temperaturbereich von rund 300 bis 900 °C durchgeführt werden. Im allgemeinen wird dabei ein Trägergasstrom, beispielsweise ein Edelgas, vorzugsweise Helium oder Argon oder Neon, über das Probenmaterial geleitet, so daß die Pyrolyse des Probenmaterials geeigneterweise im Trägergasstrom des Gaschromatographen durchgeführt wird. Beim erfindungsgemäßen Verfahren kann aber auch sauerstofffreier Stickstoff oder Wasserstoff als Trägergas eingesetzt werden.

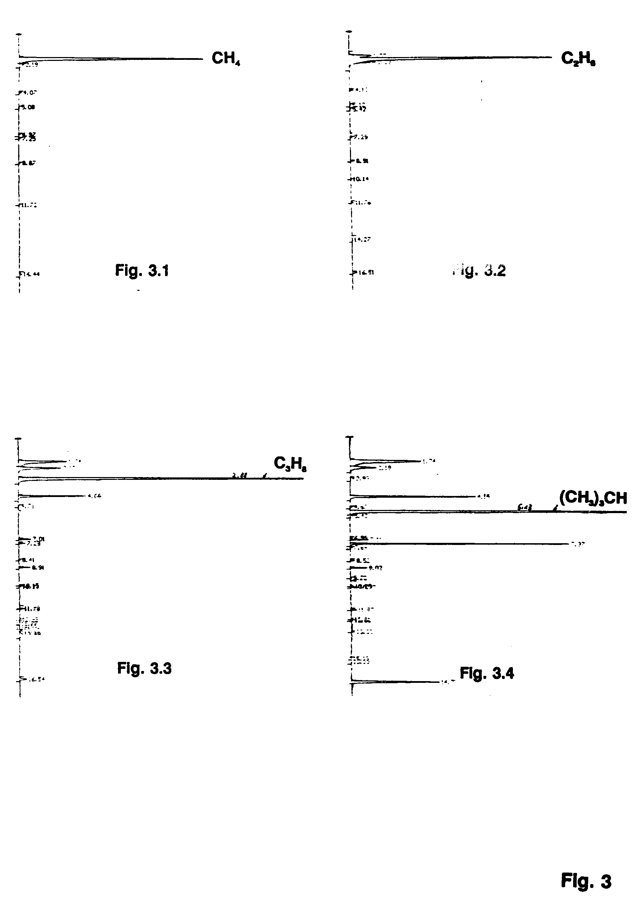

Fig. 3 gibt die Pyrolyse-Gaschromatogramme der gleichen, bereits bezüglich Abb. 2 beschriebenen Proben wieder; geeigneterweise wurde hier eine PLOT-Säule 2 (25 m Länge, 0,32 mm innerer Durchmesser, Schicht aus Al₂O₃/KCl, Schichtdicke 5 µm, Chrompack GmbH, Frankfurt/Main) eingesetzt. Mit der hier gewählten Pyrolyseanordung, vgl. auch das erfindungsgemäße Beispiel 1, kann schon bei rund 500 °C der an Silizium gebundene Organorest vollständig abgespalten werden, das entsprechende Alkan ist auch hier die einzige Komponente, die als Pyrolyseprodukt in größerer Menge gebildet wird.

Darüber hinaus kann das erfindungsgemäße Verfahren auch bei einer Pyrolysetemperatur von rund 800 °C durchgeführt werden, die Zahl und Menge sonstiger kleinerer Bruchstücke steigt dabei in der Regel nicht wesentlich an.

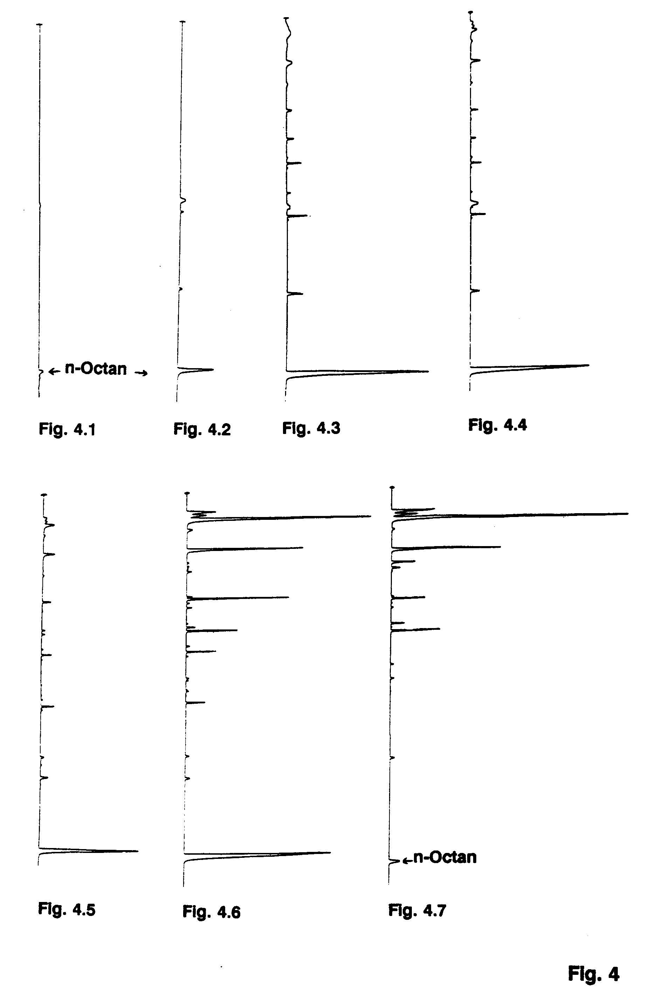

Fig. 4 zeigt beispielsweise den Temperatureinfluß für das thermisch weniger beständige n-Octyltriethoxysilan auf Beton. Als Pyrolysetemperatur wurde insbesondere der Bereich von 300 bis 900 °C untersucht: Unter 500 °C findet nur eine schwache Abspaltung des Octylrestes (der als Octan auftritt) statt. Oberhalb von 900 °C wird dieser Rest weitgehend zersetzt. Der bevorzugte Bereich für die Pyrolysetemperatur liegt auch hier zwischen 500 und 800 °C, das auf Beton aufgebrachte Octyltriethoxysilan sowie seine auf oder in der Betonoberfläche gebildeten und verankerten Hydrolyse-, Kondensations- und Vernetzungsprodukte zersetzen sich dabei bevorzugt zu n-Octan.

Das erfindungsgemäße Verfahren kann auch an anderen, mit Organosiliziumverbindungen behandelten anorganischen Materialien durchgeführt werden: beispielsweise an mineralischen Produkten, Sandstein, Kalkstein, ton- oder silikathaltigen Materialien, Ziegelstein, Mörtel, Pigmenten, aluminiumoxidhaltigen Materialen, titanoxidhaltigen Materialien, magnesiumoxidhaltigen Materialien oder entsprechenden Stoffen, Quarzprodukten oder Quarzpulver, Glasprodukten - wie Glasmatten, Glasfasern, Glasperlen, Mikroglasperlen, Glaspulver, optischen Gläsern oder Isoliermaterialien wie Glaswolle oder Steinwolle, aber auch an Füllstoffen oder Metallen oder Legierungen. Diese Materialien können in fester, duktiler, pulverförmiger, körniger und/oder glasartig oxidischer, hydroxidischer, carbonatischer, metallischer und/oder silikatisch dichter und/oder poröser Form vorliegen.

Für die Behandlung zuvor beispielhaft aufgeführter Materialien sind auch thermisch und chemisch instabilere Silane einsetzbar, wie z. B. 3-Methacryloxypropyl-trimethoxysilan (MEMO) und 3-Aminopropyl-trimethoxysilan (AMMO). Bei diesen Verbindungen wurden im bevorzugten Temperaturbereich von 500 bis 800 °C nicht hauptsächlich kleine C₁-C₃-Bruchstücke gebildet, sondern auch charakteristische Höhersieder, die erstaunlicherweise während des Pyrolysevorgangs unter den erfindungsgemäßen Verfahrensbedingungen erhalten bleiben.

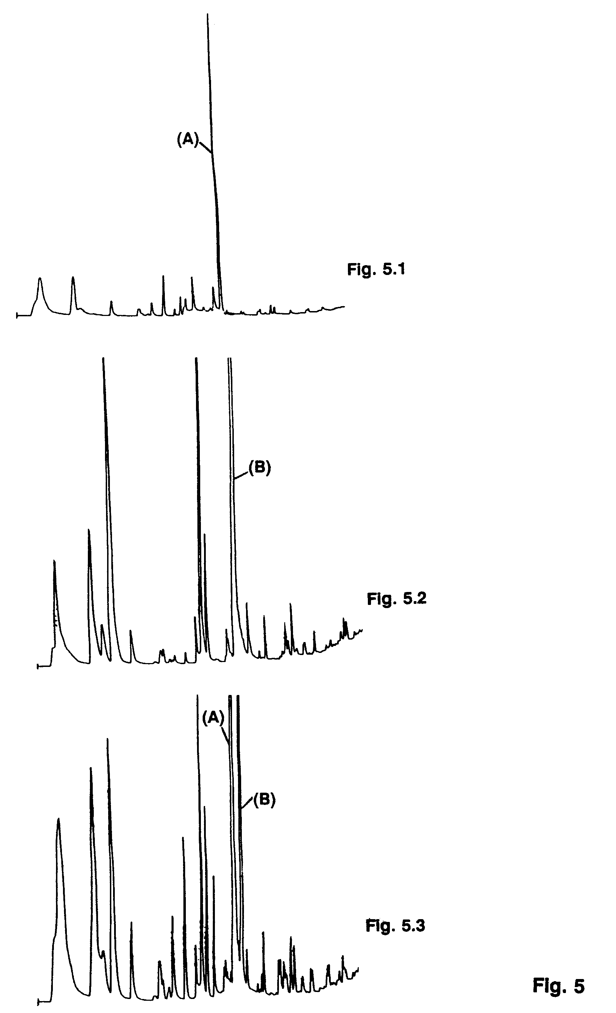

Fig. 5.1 zeigt beispielsweise das Pyrolyse-Gaschromatogramm eines mit 1 % 3-Aminopropyl-triethoxysilan (AMEO: H₂N-(CH₂)₃Si(OC₂H₅)₃) dotierten Betonpulvers (Die Hauptkomponente A besitzt eine Retentionszeit von 9,133 Minuten). Fig. 5.2 zeigt das Pyrolyse-Gaschromatogramm einer mit 0,5 % 3-Methacryloxypropyl-trimethoxysilan (MEMO: CH₂=C(CH₃)COO(CH₂)₃Si(OCH₃)₃) beschichteten Betonpulverprobe (Die Retentionszeit der hier entstandenen Hauptkomponente B beträgt 8,743 Minuten) und Fig. 5.3 zeigt das Untersuchungsdigramm einer Mischung von AMEO und MEMO auf Beton. Die Zuordnung der Pyrolyseprodukte zu den jeweils im Probenmaterial für die Hydrophobierung bzw. Modifizierung eingesetzten Organosiliziumverbindungen ist in der Regel unschwer zu treffen. Auch der Alkoxyrest des ursprünglich zur Beschichtung eingesetzten Silans, beispielsweise eines Organotrimethoxy- bzw. Organotriethoxysilans, kann an Hand des sich als kleine Nebenkomponente bildenden Methanol- bzw. Ethanolpeaks identifiziert werden.

Beim erfindungsgemäßen Verfahren können die Organosiliziumverbindungen, d. h. die Hydrophobierungs- bzw. Beschichtungs- oder Modifizierungsmittel, anhand von Komponenten, die unter den vorherrschenden Pyrolysebedingungen bevorzugt gebildet werden, nicht nur identifiziert, sondern auch quantitativ bestimmt werden.

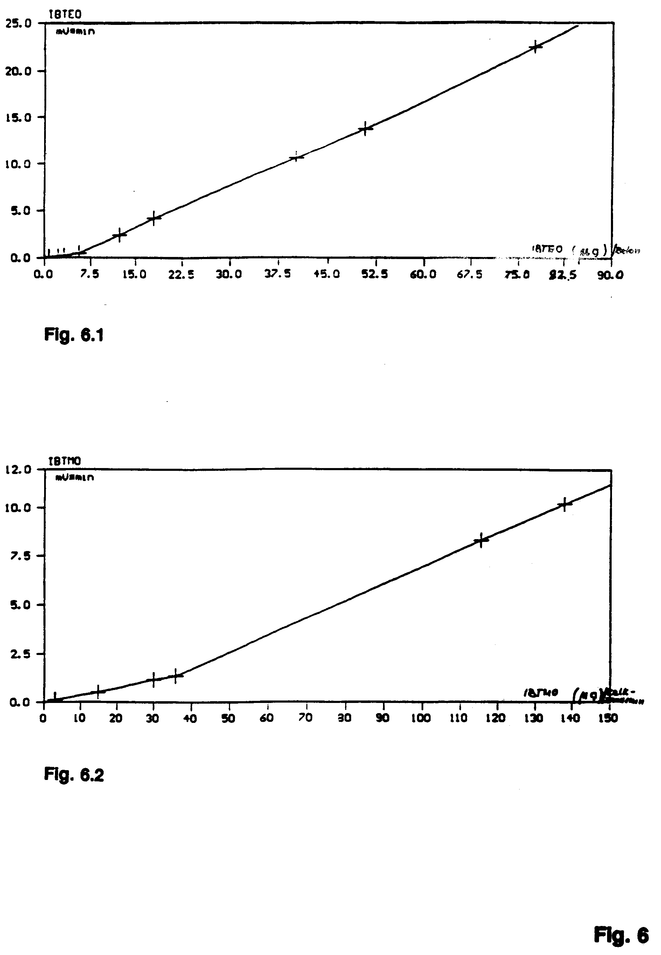

Fig. 6 zeigt die Kalibrierkurven für Isobutyltriethoxysilan (IBTEO) auf Beton und für Isobutyltrimethoxysilan (IBTMO) auf Kalksandstein. Ordinate ist die Detektoranzeige in mV · min, und auf der Abszisse sind die Mikrogramm an Silan, die zur Pyrolyse kamen, eingetragen. Beispielsweise entspräche eine Einwaage von 3 mg eines Betons, der 2,57 % an Silan enthielte, einer absoluten Menge von 77 µg Silan und würde ein Detektorsignal von 22,3 mV · min für das charakteristische Pyrolyseprodukt Isobutan liefern, und 30 mg eines Betons mit 0,01 % IBTEO (= 3 µg IBTEO) ergäben ein Detektorsignal von 1,8 mV · min (beide Punkte sind in der oberen Kurve eingezeichnet). Anhand der Fläche des für das verwendete Silan charakteristischen Pyrolysepeaks kann also die Bestimmung der Silankonzentration in einem weitem Bereich durch Variation der Einwaage erfolgen.

Üblicherweise liegt die Konzentration der zur Hydrophobierung bzw. Modifizierung auf anorganische Materialien aufgebrachten bzw. hier eingetragenen Organosiliziumverbindungen oder deren Folge- bzw. Hydrolyse- oder Kondensationsprodukte bei rund 5 bis 0,01 Gew.-%, bezogen auf das vorliegende Probenmaterial, die Konzentrationen können davon aber auch nach oben oder nach unten abweichen.

Die Wiederholstandardabweichung der Bestimmung von Bautenschutzmitteln liegt bei etwa 0,12, die Wiederholbarkeit bei 0,34 und der Variationskoeffizient bei einem Mittelwert von beispielsweise 1,5 % Hydrophobierungsmittel bei 8 %.

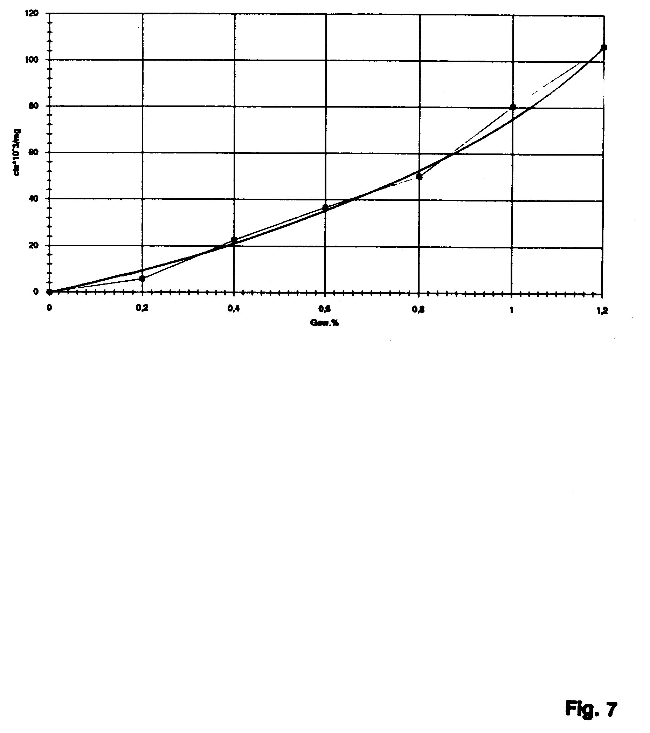

Eine weitere Kalibrierkurve (3-Methacryloxypropyl-trimethoxysilan, MEMO) auf Quarzpulver (Cristobalit) zeigt Fig. 7, bei der die von einem Integrator gemessenen Peakflächenwerte in counts/mg Einwaage gegen die Gewichtsprozente aufgetragen sind.

Es wurde auch gefunden, daß das anorganische Material, auf das die Silane aufgebracht sind (bei Hydrophobierungen z. B. Beton, Kalksandstein, Vormauerziegel, bei Beschichtungen z. B. Quarz, Glas-, Metallpulver, Aluminiumoxid, Titandioxid u. a.), die Pyrolyse beeinflussen kann. Deshalb werden die Kalibrierkurven geeigneterweise mit einem weitgehend vergleichbaren Material, vorzugsweise mit dem gleichen Material aufgestellt.

Man kann beim erfindungsgemäßen Verfahren den Substrateinfluß auf die Pyrolyse weitgehend eliminieren, indem vorzugsweise ein Teil des Probenmaterials pyrolysiert wird, der Blindwert des Pyrolyserückstands erfindungsgemäß bestimmt, der Pyrolyserückstand danach mit einer organosiliziumhaltigen Vergleichsverbindung behandelt und nochmals untersucht wird.

In Anbetracht der beim erfindungsgemäßen Verfahren zur Pyrolyse kommenden Probenmengen sollte die Probe so homogen wie möglich sein - gutes Durchmischen bei Pulvern ist deshalb zu bevorzugen. Für die Pyrolyse werden beim erfindungsgemäßen Verfahren vorzugsweise 0,1 mg bis 500 mg, besonders vorzugsweise 0,5 mg bis 100 mg, ganz besonders vorzugsweise 1 mg bis 50 mg, Probenmaterial, das geeigneterweise einen Gehalt an Organosiliziumverbindungen von 5 bis 0,01 Gew.-% aufweist, eingesetzt.

Für die Bestimmung der Eindringtiefe in ein poröses Material, beispielsweise bei Baustoffen wie Beton oder Kalksandstein, wird geeigneterweise so verfahren, daß einem Probenkörper an der Oberfläche und/oder an verschiedenen Tiefen durch vorsichtiges Schleifen, Bohren oder Fräsen Proben entnommen und nach den Lehren der vorliegenden Erfindung untersucht werden.

Der Probekörper kann dafür auch in eine geeignete Spannvorrichtung eingespannt, beispielsweise mit einem Mikro-Trennschleifer (z. B. Diamant-Trennscheibe, Durchmesser 10 - 30 mm, Schleifbreite 0,1 - 0,2 mm) oder einem Mikrobohrer (Diamant-Bohrer, Durchmesser ≦ 1 mm) an der Oberfläche sowie auch in den zu prüfenden Tiefen des Prüfkörpers (z. B. in Tiefen von 1 mm, 2 mm, 4 mm, 8 mm, oder auch häufiger und bei geringeren oder größeren Abständen) Prüfmaterial so abgetragen werden, daß vorzugsweise etwa 10 - 100 mg Pulver anfallen, und anschließend kann nach dem erfindungsgemäßen Verfahren die Untersuchung des Probenmaterials erfolgen.

Das für jede Eindringtiefe separat aufgefangene pulverförmige Material wird vorzugsweise von zu groben Bruchstücken befreit (um die Homogenität zu sichern), gut durchmischt und eine Teilprobe davon analysiert. Beim Schleifen oder Bohren oder Fräsen sollte darauf geachtet werden, daß die Umdrehungsgeschwindigkeit der Schleifscheibe, des Bohrers bzw. der Fräse möglichst niedrig gehalten wird, um ein zu starkes Erhitzen des abzuschleifenden Materials zu vermeiden. Aus den mittels Pyrolyse-Gaschromatographie ermittelten Wirkstoffkonzentrationen und den Tiefen, aus denen die Proben genommen wurden, kann ein Wirkstoffprofil (Konzentration gegen Tiefe aufgetragen) konstruiert werden.

Da es für die Qualität eines Bautenschutzes entscheidend ist, ob die Hydrophobierungsmittel nur physikalisch auf den äußeren und inneren Oberflächen lagern und damit auswaschbar sind oder ob sie chemisch an die Oberfläche gebunden sind und dadurch eine dauerhafte Imprägnierung gewährleisten, ist es vorteilhaft, die jeweiligen Proben wie zuvor beschrieben zu analysieren und zusätzlich Teile der Probe vor der Pyrolyse mit einem geeigneten Lösemittel, beispielsweise n- oder i-Pentan, n- oder i- oder cyclo-Hexan, n- oder i-Heptan, n- oder i-Octan oder Toluol, Xylole oder Methanol, Ethanol oder Methylenchlorid oder Trifluortrichlorethan (R 113) oder andere Chlor- oder Fluorchlorkohlenwasserstoffe, zu extrahieren, zu trocknen und dann zu analysieren, vgl. hierzu auch Beispiel 3.

Beim erfindungsgemäßen Verfahren kann somit auch eine zusätzliche Information über den Bindungscharakter von Hydrophobierungs- bzw. Modifizierungsmitteln nach der Behandlung eines anorganischen Materials erhalten werden, wenn man geeigneterweise mindestens eine Teilprobe des Probematerials vor der Pyrolyse mit einem Lösemittel extrahiert, trocknet und danach gesondert pyrolysiert und analysiert.

Das erfindungsgemäße Verfahren ermöglicht in einfacher und wirtschaftlicher Weise Silan-haltige Hydrophobierungsmittel und Modifizierungsmittel auf anorganischen Materialien, insbesondere Baustoffen, nachzuweisen und zu identifizieren und weitestgehend zuverlässig zu quantifizieren. Darüber hinaus ermöglicht das erfindungsgemäße Verfahren in einfacher und zuverlässiger Weise auch die Bestimmung der Eindringtiefe der mit Organosiliziumverbindungen entsprechend hydrophobierten bzw. modifizierten Materialien.

Die Erfindung wird durch die nachfolgenden Beispiele sowie die Abbildungen Fig. 1 bis 10 näher erläutert:

### Beispiel 1

Von einer mit Isobutyltrimethoxysilan (IBTMO) hydrophobierten (120 g/m²) Betonprobe (die aus einer hydrophobierten Betonwand herausgebohrt wurde und als zylindrischer Körper vorlag) wurden mit einer Diamanttrennscheibe (Scheibendicke 0,1 mm) von der Oberfläche, 4,5 mm unterhalb, 8 mm unterhalb und 18 mm unterhalb der Oberfläche jeweils ca. 20 mg Betonpulver herausgeschliffen, wobei stets darauf geachtet wurde, daß die Umdrehungszahl der Trennscheibe niedrig blieb, um eine vorzeitige Zersetzung des Hydrophobierungsmittels zu vermeiden, und daß nicht etwa in den Beton eingeschlossene Kieselsteine (die kaum Hydrophobierungsmittel aufnehmen), sondern einigermaßen repräsentative Betonmasse an verschiedenen Stellen der jeweiligen Tiefe abgefräst wurden. Die jeweiligen Proben aus den verschiedenen Tiefen wurden einzeln gründlich gemischt und größere Brocken, die Inhomogenitäten bewirken könnten, aussortiert und verworfen.

Ca. 1 - 5 mg des abgefrästen Pulvers wurden in ein in den Pyrolyzer PYR-2 der Shimadzu Europa GmbH passendes Platinschiffchen genau eingewogen und bei 700 °C pyrolysiert, indem eine Einschubstange mit dem Mikroschiffchen in die 700 °C heiße Zone geschoben wurde und die schlagartig entstehenden Pyrolyseprodukte sofort mit dem Trägergas Helium on-line in die gaschromatographische Trennsäule gespült, dort getrennt und von einem Flammenionisationsdetektor angezeigt wurden.

Trennsäule: 10 m Quarzkapillare PoraPLOT Q, Innendurchmesser: 0,32 mm, Schichtdicke: 10 µm, Chrompack GmbH, Frankfurt/Main.
- Temperaturprogramm:: 60 °C/2 min isotherm,
10 °C/min bis 120 °C, 9 min isotherm.

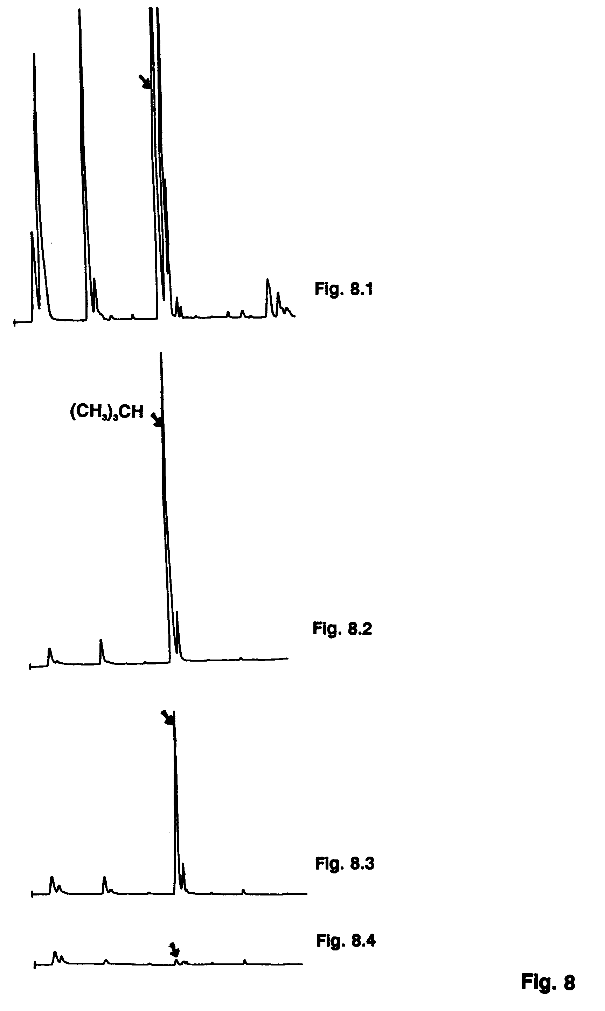

Die entsprechenden Gaschromatogramme zeigt Fig. 8. Aus den Peakflächenwerten (counts oder mV · min), bezogen auf die Einwaage, wurden die Gewichtsprozente Wirkstoff auf Beton an Hand einer Kalibrierkurve berechnet: Oberfläche 1,05 %, 4,5 mm Tiefe: 0,36 %, 8 mm Tiefe: 0,14 %, 18 mm Tiefe: < 0,01 %.

Die Kalibrierkurve (z. B. Fig. 6 und 7) gewinnt man auf folgende Weise: Etwa 200 mg eines geeigneten Betons werden abgefräst. Nach Überprüfung, daß der Beton unter den Bedingungen der Pyrolyse-Gaschromatographie keine die Auswertung störenden organischen Bestandteile abgibt, werden jeweils 20 mg davon in ein Rollrandgläschen eingewogen, mit unterschiedlichen Konzentrationen einer ethanolischen (Organoethoxysilane) oder methanolischen (Organomethoxysilane) Lösung des betreffenden Silans versetzt, verschlossen und 24 Stunden bei Raumtemperatur stehen gelassen. Danach wird das Lösemittel (Methanol oder Ethanol) im Stickstoffstrom abgeblasen (Abzug) und der nun trockene Rückstand 30 Minuten im Trockenschrank offen bei 70 °C gehalten, um restliche Alkoholspuren auszutreiben. Die einzelnen Proben werden pyrolysiert und die Peakflächenwerte des betreffenden Hauptpeaks gegen die Organoalkoxysilanmenge (µg, berechnet aus aufgegebener Menge und Einwaage, Fig. 6) oder die Peakflächenwerte pro mg Einwaage gegen die Organoalkoxysilankonzentration (Gew.-% Organoalkoxysilan auf Beton, Fig. 7) aufgetragen und die Kurve konstruiert.

### Beispiel 2

Um festzustellen, ob das Hydrophobierungsmittel chemisch gebunden vorliegt oder nur physikalisch adsorbiert ist, wurde wie folgt vorgegangen:

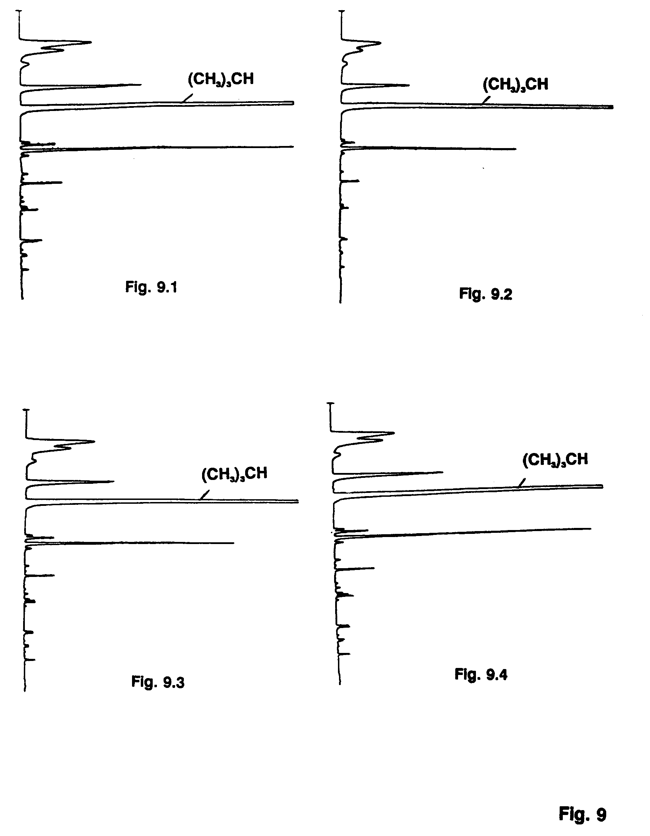

Aus einer Tiefe von 3 mm eines mit Isobutyltriethoxysilan hydrophobierten Betons wurden 150 mg vorsichtig herausgefräst und 5,362 mg bzw. 4,111 mg davon analog Beispiel 1 analysiert (Fig. 9.1 und 9.2).

Als Trennsäule dienten hier 25-m-Quarzkapillare PoraPLOT Al₂O₃/KCl, Innendurchmesser: 0,32 mm, Schichtdicke: 5 µm, der Chrompack GmbH, Frankfurt/Main.
- Temperaturprogramm:: 60 °C/2 min isotherm,
12 °C/min bis 180 °C, 12 min isotherm.
An Wirkstoff wurden 0,40 Gew.% gefunden.

Parallel dazu wurden 125 mg des gleichen Betonpulvers mit 40 ml n-Heptan (Kp 98 °C) 3,5 Stunden in einem Mikro-Soxhlet extrahiert und danach 1 Stunde im Vakuumtrockenschrank bei 90 °C und eine weitere Stunde im normalen Trockenschrank bei 80 °C von restlichem Heptan befreit. Von diesem extrahierten Beton wurden ebenfalls 2 pyrolyse-gaschromatographische Analysen durchgeführt (Einwaagen: 4,821 bzw. 4,943 mg, Fig. 9.3 und 9.4). Ergebnis: 0,39 Gew.% Wirkstoff, d. h. 97,5 % des Isobutyltriethoxysilans sind demnach an Beton gebunden und deshalb nicht extrahierbar, und nur 2,5 % liegen physikalisch adsorbiert vor. Entsprechend diesem Ergebnis unterscheiden sich die Pyrolyse-Gaschromatogramme der extrahierten Proben (Fig. 9.3 und Fig. 9.4) kaum von denen der nicht extrahierten Proben (Fig. 9.1 und 9.2).

### Beispiel 3

Untersuchung mineralischer pulverförmiger Materialien:

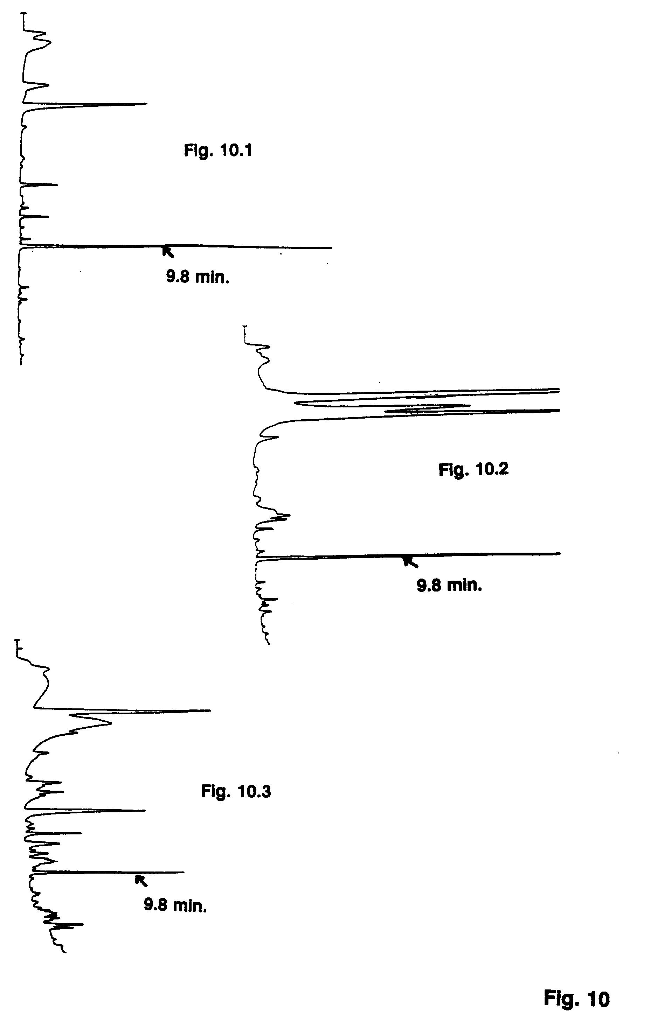

In Abhängigkeit von der erwarteten Silankonzentration werden ca. 1 mg (bei > 2 Gew.%) bis 50 mg (bei < 0,02 Gew.%) genau eingewogen und wie beschrieben pyrolysiert. Da im Gegensatz zu den Bautenschutzmitteln, bei denen es sich vorwiegend um Alkylalkoxysilane handelt, für die Beschichtung vorwiegend organofunktionelle Alkoxysilane wie z. B. Acryloxypropyl-, 3-Methacryloxypropyl-, Glycidoxypropyl-, 3-Aminopropyl-trialkoxysilane eingesetzt werden, die eine chemisch und thermisch weniger stabile Organogruppe tragen, ist auch der Einfluß des Substrats auf die Pyrolyse stärker. Dies zeigen Fig. 10.1 bis 10.3, wo das gleiche Silan (3-Aminopropyl-trimethoxysilan) jeweils auf Betonpulver (Fig. 10.1), auf Aluminiumoxidhydrat Al₂O₃ · 3 H₂O (Fig. 10.2) bzw. auf Talkum (Fig. 10.3) aufgebracht worden war. Das charakteristische größte höhersiedende Pyrolyseprodukt (Retentionszeit 9,8 Minuten) tritt zwar in jedem Fall auf, doch sind tiefersiedende Spaltprodukte je nach Aktivität des Substrats in unterschiedlichem Maße vertreten: Die Komponente mit der Retention 7,2 Minuten z. B. (n-Propanol) tritt vorwiegend bei Talkum auf, bei dem aktiveren Aluminiumoxid aber nur in Spuren. Aus diesem Grund empfiehlt es sich, wie folgt vorzugehen, wenn solche Silane vorliegen könnten:

Ein Teil der Probe wird bei 700 °C pyrolysiert. Einige Milligramm davon werden zur Kontrolle, ob die pyrolysierbaren Anteile vollständig zersetzt worden sind, erneut einer Pyrolyse, aber bei 500 °C, unterworfen. Falls dieses Material erwartungsgemäß nur einen vernachlässigbar kleinen Blindwert liefert, wird es mit einer alkoholischen Lösung des vermuteten Silans beschichtet, 24 Stunden zur Reaktion stehen gelassen, der Alkohol abgetrieben und das Ganze noch 30 Minuten bei 70 °C im Trockenschrank getrocknet. Bei 500 °C wird dann pyrolysiert und das Pyrolyse-Gaschromatogramm mit dem der unbekannten Probe verglichen. Bei Übereinstimmung werden dann auf das bei 700 °C pyrolysierte Material unterschiedliche Silanmengen aufgebracht und, wie bereits beschrieben, eine Kalibrierkurve aufgestellt. Auf diese Weise entspricht das Substrat vollständig dem der zu untersuchenden Probe, und Fehlinterpretationen aufgrund von Substrateinflüssen können vermieden werden.

### Kurze Beschreibung der Abbildungen:

- Fig. 1:: Herkömmliche gaschromatographische Untersuchung von Reaktionsprodukten, die bei der Pyrolyse von verschiedenen Alkyltriethoxysilanen auf Beton anfallen (Fig. 1.1 bis 1.4)
- Fig. 1.1:: Pyrolyse-Gaschromatogramm (GC) von 0,4 Gew.-% Methyltriethoxysilan auf Beton
- Fig. 1.2:: Pyrolyse-GC von 0,4 Gew.-% Ethyltriethoxysilan auf Beton
- Fig. 1.3:: Pyrolyse-GC von 0,4 Gew.-% Propyltriethoxysilan auf Beton
- Fig. 1.4:: Pyrolyse-GC von 0,4 Gew.-% Isobutyltriethoxysilan auf Beton
- Fig. 2:: Gaschromatographische Untersuchung an einer PLOT-Säule 1 nach dem erfindungsgemäßen Verfahren - Pyrolyse von verschiedenen Alkyltriethoxysilanen auf Beton (Fig. 2.1 bis 2.4)
- Fig. 2.1:: Pyrolyse-GC von 0,4 Gew.-% Methyltriethoxysilan auf Beton
- Fig. 2.2:: Pyrolyse-GC von 0,4 Gew.-% Ethyltriethoxysilan auf Beton
- Fig. 2.3:: Pyrolyse-GC von 0,4 Gew.-% Propyltriethoxysilan auf Beton
- Fig. 2.4:: Pyrolyse-GC von 0,4 Gew.-% Isobutyltriethoxysilan auf Beton
- Fig. 3:: Gaschromatographische Untersuchung an einer PLOT-Säule 2 nach dem erfindungsgemäßen Verfahren - Pyrolyse von verschiedenen Alkyltriethoxysilanen auf Beton (Fig. 3.1 bis 3.4)
- Fig. 3.1:: Pyrolyse-GC von 0,4 Gew.-% Methyltriethoxysilan auf Beton
- Fig. 3.2:: Pyrolyse-GC von 0,4 Gew.-% Ethyltriethoxysilan auf Beton
- Fig. 3.3:: Pyrolyse-GC von 0,4 Gew.-% Propyltriethoxysilan auf Beton
- Fig. 3.4:: Pyrolyse-GC von 0,4 Gew.-% Isobutyltriethoxysilan auf Beton
- Fig. 4:: Gaschromatographische Untersuchung nach dem erfindungsgemäßen Verfahren - Bildung von n-Octan bei der Pyrolyse von n-Octyltriethoxysilan auf Beton in Abhängigkeit von der Pyrolysetemperatur im Bereich von 300 bis 900 °C (Fig. 4.1 bis 4.7)
- Fig. 4.1:: Pyrolyse-GC - Pyrolysetemperatur 300 °C
- Fig. 4.2:: Pyrolyse-GC - Pyrolysetemperatur 400 °C
- Fig. 4.3:: Pyrolyse-GC - Pyrolysetemperatur 500 °C
- Fig. 4.4:: Pyrolyse-GC - Pyrolysetemperatur 600 °C
- Fig. 4.5:: Pyrolyse-GC - Pyrolysetemperatur 700 °C
- Fig. 4.6:: Pyrolyse-GC - Pyrolysetemperatur 800 °C
- Fig. 4.7:: Pyrolyse-GC - Pyrolysetemperatur 900 °C
- Fig. 5:: Gaschromatographische Untersuchungen nach dem erfindungsgemäßen Verfahren - Pyrolyse von Silanen mit unterschiedlichen organofunktionellen Gruppen (Fig. 5.1 bis 5.3)
- Fig. 5.1:: Pyrolyse-GC von 1 Gew.-% 3-Aminopropyltriethoxysilan (AMEO) auf Beton (Pyrolysetemperatur: 500 °C, Einwaage: 3,050 mg, PLOT-Säule: Pora PLOT Q)
- Fig. 5.2:: Pyrolyse-GC von 0,5 % 3-Methacryloxypropyltrimethoxysilan (MEMO) auf Beton (Pyrolysetemperatur: 500 °C, Einwaage: 5,130 mg, PLOT-Säule: Pora PLOT Q)
- Fig. 5.3:: Pyrolyse-GC eines Gemisches von 1 Gew.-% AMEO auf Beton und 0,5 Gew.-% MEMO auf Beton. Einwaage 4,5 mg AMEO-dotierter und 3,1 mg MEMO-dotierter Beton (Pyrolysetemperatur: 500 °C, PLOT-Säule: Pora PLOT Q)
- Fig. 6:: Beispiele für Kalibrierkurven
- Fig. 6.1:: Kalibrierkurve für Isobutyltriethoxysilan (IBTEO) auf Beton
- Fig. 6.2:: Kalibrierkurve für Isobutyltrimethoxysilan (IBTMO) auf Kalksandstein
- Fig. 7:: Kalibrierkurve für 3-Methacryloxypropyltrimethoxysilan (MEMO) auf Quarzpulver (Cristobalit)
- Fig. 8:: Bestimmung eines Tiefenprofils nach dem erfindungsgemäßen Verfahren - an der Oberfläche, in 4,5 mm Tiefe, in 8 mm Tiefe und in 18 mm Tiefe eines mit Isobutyltrimethoxysilan (IBTMO) behandelten Betons.
- Fig. 8.1:: an der Oberfläche (Einwaage: 1,524 mg)
- Fig. 8.2:: in 4,5 mm Tiefe (Einwaage: 2,471 mg)
- Fig. 8.3:: in 8 mm Tiefe (Einwaage: 3,684 mg)
- Fig. 8.4:: in 18 mm Tiefe (Einwaage: 4,109 mg)
- Fig. 9:: Auswirkung der Extraktion auf die Hydrophobierung von Beton - Untersuchungen unter Verwendung einer Pora PLOT-Säule mit Al₂O₃/KCl (Fig. 9.1 bis 9.4)
- Fig. 9.1:: Mit Isobutyltriethoxysilan behandelter Beton (in 3 mm Tiefe), vor der Extraktion (Einwaage: 5,362 mg)
- Fig. 9.2:: Mit Isobutyltriethoxysilan behandelter Beton (in 3 mm Tiefe), vor der Extraktion (Einwaage: 4,111 mg)
- Fig. 9.3:: Mit Isobutyltriethoxysilan behandelter Beton (in 3 mm Tiefe), nach erschöpfender Extraktion mit n-Heptan (Einwaage: 4,821 mg)
- Fig. 9.4:: Mit Isobutyltriethoxysilan behandelter Beton (in 3 mm Tiefe), nach erschöpfender Extraktion mit n-Heptan (Einwaage: 4,943 mg)
- Fig. 10:: Pyrolyse-GC nach dem erfindungsgemäßen Verfahren - 3-Aminopropyltrimethoxysilan (AMEO) auf verschiedenen anorganischen Materialien (Fig. 10.1 bis 10.3)
- Fig. 10.1:: 0,5 Gew.-% AMEO auf Beton (Pyrolysetemperatur: 500 °C, Einwaage: 6,229 mg, PLOT-Säule: 10 m Pora PLOT Q)
- Fig. 10.2:: 0,5 Gew.-% AMEO auf Aluminiumoxidhydrat (Al₂O₃ · 3 H₂O) (Pyrolysetemperatur: 500 °C, Einwaage: 11,752 mg, PLOT-Säule: 10 m Pora PLOT Q)
- Fig. 10.3:: 0,5 Gew.-% AMEO auf Talkum (Pyrolysetemperatur: 500 °C, Einwaage: 5,351 mg, PLOT-Säule: 10 m Pora PLOT Q)

## Patentansprüche

1. Verfahren zur Untersuchung von anorganischen Materialien, die mit siliziumorganischen Verbindungen behandelt sind,
dadurch gekennzeichnet,
daß das Probenmaterial innerhalb weniger Sekunden pyrolysiert wird und die Pyrolyseprodukte on-line gaschromatographisch an einer PLOT-Säule (porous layer open-tubular -Säule, d. h. Kapillarsäule mit poröser Schicht) analysiert werden.

2. Verfahren nach Anspruch 1,
dadurch gekennzeichnet,
daß die Identifizierung der Organosiliziumverbindungen anhand von Komponenten, die unter den vorherrschenden Pyrolysebedingungen bevorzugt gebildet werden, durchgeführt wird.

3. Verfahren nach den Ansprüchen 1 und 2,
dadurch gekennzeichnet,
daß die quantitative Bestimmung der Organosiliziumverbindungen anhand von Komponenten, die unter den vorherrschenden Pyrolysebedingungen bevorzugt gebildet werden, durchgeführt wird.

4. Verfahren nach den Ansprüchen 1 bis 3,
dadurch gekennzeichnet,
daß die Pyrolyse des Probenmaterials im Temperaturbereich zwischen 800 °C und 500 °C durchgeführt wird.

5. Verfahren nach den Ansprüchen 1 bis 4,
dadurch gekennzeichnet,
daß die Pyrolyse des Probenmaterials im Trägergasstrom des Gaschromatographen durchgeführt wird.

6. Verfahren nach den Ansprüchen 1 bis 5,
dadurch gekennzeichnet,
daß die Pyrolysedauer des Probenmaterials weniger als 10 Sekunden beträgt.

7. Verfahren nach den Ansprüchen 1 bis 6,
dadurch gekennzeichnet,
daß für die Pyrolyse 0,1 mg bis 500 mg Probenmaterial eingesetzt werden.

8. Verfahren nach den Ansprüchen 1 bis 7,
dadurch gekennzeichnet,
daß für die Pyrolyse Probenmaterial mit einem Gehalt von 5 bis 0,01 Gew.-% an Organosiliziumverbindungen eingesetzt wird.

9. Verfahren nach den Ansprüchen 1 bis 8,
dadurch gekennzeichnet,
daß für die gaschromatographische Untersuchung der Pyrolyseprodukte PLOT-Säulen, die Aluminiumoxid, desaktiviert durch KCl oder Na₂SO₄, oder Silicagel oder graphitierten thermischen Ruß oder organische Polymere auf Basis Styrol/Divinylbenzol als feste poröse Schicht enthalten, eingesetzt werden.

10. Verfahren nach den Ansprüchen 1 bis 9,
dadurch gekennzeichnet,
daß einem Probenkörper an der Oberfläche und/oder in verschiedenen Tiefen durch vorsichtiges Schleifen, Bohren oder Fräsen Proben entnommen und untersucht werden.

11. Verfahren nach den Ansprüchen 1 bis 10,
dadurch gekennzeichnet,
daß mindestens eine Teilprobe des Probenmaterials vor der Pyrolyse mit einem Lösemittel extrahiert, getrocknet und danach gesondert pyrolysiert und analysiert wird.

12. Verfahren nach den Ansprüchen 1 bis 11,
dadurch gekennzeichnet,
daß der Substrateinfluß auf die Pyrolyse weitgehend eliminiert wird, indem ein Teil des Probenmaterials pyrolysiert wird, der Blindwert des Pyrolyserückstands bestimmt, der Pyrolyserückstand danach mit einer organosiliziumhaltigen Vergleichsverbindung behandelt und nochmals untersucht wird.
